# EUROPEAN PATENT SPECIFICATION

(11) **EP 1 138 662 B1**
(45) Date of publication and mention of the grant of the patent: **30.06.2004**
(21) Application number: 00302736.4
(22) Date of filing: 31.03.2000
(51) Int. Cl.: C07C 45/46, C07C 49/76

(54) **A process for the preparation of 4'-isobutylacetophenone**
Verfahren zur Herstellung von 4'-Isobutylacetophenon
Procédé pour la préparation de 4'-isobutylacétophénone

(43) Date of publication of application: 04.10.2001
(73) Proprietor: Council of Scientific and Industrial Research, New Delhi 110 001 (IN)
(72) Inventor: Choudary, Boyapati Manoranjan, Hyderabad 500 007 (IN); Sateesh, Mutyala, Hyderabad 500 007 (IN); Kantam, Mannepalli Lakshmi, Hyderabad 500 007 (IN); Ranganath, Kulluri Venkata Sri, Hyderabad 500 007 (IN); Raghavan, Kondapuram Vijaya, Hyderabad 500 007 (IN)
(74) Representative: Carpmaels & Ransford

(56) References cited:
- EP-A- 0 701 987
- WO-A-97/48665
- DATABASE WPI Section Ch, Week 199522 Derwent Publications Ltd., London, GB; Class B05, AN 1995-167205 XP002149028 & JP 07 089893 A (TORAY IND INC), 4 April 1995 (1995-04-04)

## Description

### Field of the invention

The present invention relates to a process for 4'-isobutylacetophenone (4-IBAP) by Friedel-Crafts acylation of isobutylbenzene. More particularly, this invention relates to a process for the preparation of 4'-isobutylacetophenone (4-IBAP) from isobutylbenzene using acetic anhydride as an acylating agent in the presence of nanocrystalline, microcrystalline and metal exchanged nano- and microcrystalline zeolite beta catalysts.

### Background of the invention

4'-isobutylacetophenone (4-IBAP) is an important intermediate for 2-(4-isobutylphenyl)propionic acid (trade name; ibuprofen), a well-known nonsteroidal anti-inflarnmatory, antipyretic and analgesic drug and also other medical drugs.

This invention particularly relates to an ecofriendly process for 4'-isobutylacetophenone (4-IBAP) from isobutylbenzene using acetic anhydride as an acylating agent and zeolite beta as catalyst dispensing the use of stoichiometric amounts of corrosive, toxic aluminium chloride and hydrogen fluoride as Friedel-Crafts reagents.

### Prior Art References

Reference may be made to a publication by Baddley et al., Journal of Chemical Society, 1956, 4943, wherein 4' -isobutylacetophenone is prepared by the Friedel-Crafts acetylation of isobutylbenzene with acetyl chloride using aluminium chloride as catalyst. Reference may be made to a US patent 3,385,886 wherein the production of ibuprofen, the first step of the process is preparation of 4' -isobutylacetophenone by the Friedel-Crafts acetylation of isobutylbenzene with acetyl chloride in the presence of aluminium of anhydrous aluminium chloride, an hazardous material that leaves large amount of solid wastes after the reaction and tedious separation process from the alumina gel to obtain the product.

Reference may be made to a Japanese patent publication (Early disclosure) No. 60[1985]-188,343, wherein 4' -isobutylacetophenone is prepared by the acetylation of isobutylbenzene using acetyl fluoride as an acetylating agent, prepared by reacting acetic anhydride with hydrogen fluoride as a catalyst, a combination of hydrogen fluoride and boron trifluoride. Reference may be made to US patents 4,981,995 and 5,068,448 wherein the production of ibuprofen, 4' -isobutylacetophenone is prepared by the Friedel-Crafts acetylation of isobutylbenzene with acetic anhydride using hydrogen fluoride. The 4' -isobutylacetophenone is an intermediate in a process for the production of ibuprofen. The draw-bracks in the above processes are hydrogen fluoride is extremely toxic, corrosive, generation of large amount of solid wastes after the reaction and need for industrially expensive equipment to work with hydrofluoric acid.

The inherent disadvantages in the use of conventional Lewis acid metal chlorides for Friedel-Crafts acylation are that they are non-regenerable and require more than stoichiometric amounts because of complexation with the carbonyl product formed.

Work-up to decompose the resultant intermediate complex by hydrolysis forms a large amount of waste product and separation is lengthy and expensive.

EP O 701 987 A1 discloses aromatic ketones and the use of zeolites in their production. WO 97/48665 discloses a process for acylation of an aromatic compound in the presence of a zeolite catalyst. Database WPI Section Ch, Week 199522; Class BO5, AN 1995-167205 XP002149028 discloses the preparation of acyl substituted aromatic compounds in the presence of beta-zeolite.

Obviously, different approaches have been employed for the preparation of 4' - isobutylacetophenone. There was therefore a need for a process for the preparation of 4' - isobutylacetophenone which is simple to operate and can be carried out in a media which are not toxic and corrosive. Moreover the catalyst should be simple to separate and reusable.

### Objects of the Invention

The main object of the present invention is a process for the preparation of 4'-isobutylacetophenone from isobutylbenzene which comprises reacting isobutyl benzene with acetic anhydride as an acylating agent in the presence of nanocrystalline, microcrystalline or metal exchanged nano- or microcrystalline zeolite beta catalysts at a temperature ranges between 60 to 165° C for 2-24 h, separating the catalyst by filtration from the reaction mixture and recovering the product by a conventional method which obviates the drawbacks as detailed above.

The particle size of nanocrystalline zeolite beta is 10nm to 100 nm and the particle size of microcrystalline zeolite beta is 1 µm to 50 µm.

Still another object of the present invention is the metal ions selected for the exchange of nano- and microcrystalline zeolite beta are Fe³⁺, Zn²⁺, Ce³⁺ and La²⁺.

Still another object of the present invention is the use of acetic anhydride as an acylating agent.

Still another object of the present invention is the use of isobutylbenzene as the reaction solvent.

Still another object of the present invention is the ratio of isobutylbenzene and acylating agent is 5:1 to 1:5.

Still another object of the present invention is the quantity of the catalyst is 10 to 50% by weight with respect to the acylating agent, acetic anhydride.

Yet another object of the present invention is the reaction is effected at a temperature in the range of 60 to 165° C for 2-12 h.

### Summary of the Invention

The novelty of the present invention lies in the use of nanocrystalline and microcrystalline and metal exchanged nano-and microcrystalline zeolite beta for the acylation of isobutyl benzene for the first time. Decrease in particle size of zeolite beta, enhances the density of acidic sites and surface area of zeolites, which are essential factors to increase the activity of acylation reaction. In fact the activity of these nano-and microcrystalline forms increases manifold over normal zeolites. As a result of this, the acylation of isobutyl benzene is effected successfully in reasonable yields for the first time. 4'-isobutylacetophenone is obtained by a simple process involving filtration of the catalyst from the reaction mixture and recovering the product by conventional methods.

### Detailed Description of the Invention

Accordingly, the present invention provides a process for the preparation of 4'-isobutylacetophenone, an important intermediate for ibuprofen, a widely used nonsteroidal anti-inflammatory drug wherein the said process comprises reacting isobutylbenzene with acetic anhydride as an acylating agent in the presence of nanocrystalline, microcrystalline and metal exchanged nano- and microcrystalline zeolite beta catalysts at a temperature ranges between 60 to 165° C for 2-24 h separating the catalyst by filtration from the reaction mixture and recovering the product by a conventional method.

The particle size of nanocrystalline and microcrystalline zeolite beta are 10nm to 100nm and 1µm to 50 µm.

In another embodiment of the present invention the metal ions selected for the exchange of nano- and microcrystalline zeolite beta are Fe³⁺, Zn²⁺, Ce³⁺ and La²⁺.

In yet another embodiment of the present invention acetic anhydride is used as an acylating agent.

In still another embodiment of the present invention the reaction is effected at a temperature in the range of 60 to 165° C for 2-12h.

In still another embodiment of the present invention the catalyst is separated by filtration from the reaction mixture.

### Scientific Explanation:

In the nano- and microcrystalline zeolite beta the density of the acidic sites increases because of increased number of broken edges resulted from the broken aluminium silicate rings. The surface area of these particles is also increased due to reduction of the particle size of zeolites. The higher density of acidic sites eventually increases number of acyl cations generated in the reaction in the electrophilic substitution of the Freidel-Crafts acylation and thus enhances activity of the reaction. Thus the higher density of acid sites present in nano-, microcrystalline, metal exchanged zeolite beta are responsible for the Friedel Crafs acylation of isobutyl benzene for the first time.

Nanocrystalline, microcrystalline and metal exchanged zeolite beta were prepared as described in example 1 and employed them in the acylation of isobutylbenzene with acetic anhydride as an acylating agent as described in examples.

The following examples are given by way of illustration of the present invention and therefore should not be construed to limit the scope of the invention.

### Example 1

### Catalyst Preparation

### a) Zeolite beta

Tetraethyl *ortho*silicate and aluminium nitrate of appropriate molar ratios to get desired ratio of Si /Al ranging from 5 to 100 were used. Water is added to tetraethylortho silicate and stirred. To this solution aluminium nitrate, nonahydrate in tetraethylammonium hydroxide solution is added dropwise by a pressure regulating funnel under stirring. After the addition, the solution is kept at 50 °C and later on kept at 135° C in an autoclave for one week for crystallization . Then the solid was filtered and air dried. The resultant solid was calcined at 500 °C.

### b) Microcrystalline zeolite beta-I

Microcrystalline zeolite beta-I was obtained by mechanical disintegration of the zeolite beta obtained as described above (1µm -10µm, 95%).

### c) Microcrystalline zeolite beta-Π

Microcrystalline zeolite beta was synthesised with different particle size (5µm to 50 µm, 85%) by decreasing ageing time to 48 hours instead of one week during the synthesis of zeolite beta according to the above procedure.

### d) Nanocrystalline zeolite beta

Nanocrystalline zeolite beta was synthesised with different particle size (10 nm to 100 nm) from the homogenised solution prepared in the first step of zeolite beta which is kept for crystallisation at different times by decreasing ageing time to control the nucleation growth of zeolite during the synthesis. Then the solid were separated by centrifugation and resultant solid was washed with distilled water and dried at 100 °C.

### e) Metal exchanged zeolite beta

10 g of zeolite beta as synthesised or microcrystalline zeolite beta having Si/Al= 15 was subjected to an ion-exchange procedure by stirring with 1wt% to 10wt% metal chloride (Ce³⁺, Fe³⁺, Zn²⁺ and La²⁺) solution at 80 °C for 6 hours. The resultant zeolite was washed with deionised water and dried at 120 °C. After that the metal exchanged zeolite was calcined at 500 °C for 6 hours.

### f) H⁺-exchanged zeolite beta

Zeolite beta is added to 1 Molar NH₄Cl solution (10ml/g zeolite), stirred at 60°C for 6 hours and the resultant solid was washed with deionised water and dried at 120° C. After that ammonium exchanged zeolite was calcined at 500. °C to get H⁺-exchanged zeolite beta.

### Comparative Example 2

A mixture of isobutylbenzene (40 mmol), acetic anhydride (10 mmol) and zeolite beta catalyst ( 0.5 g) were stirred in a round bottomed flask (50 ml) under nitrogen atmosphere at 130 °C temperature. After completion of the reaction (followed by G. C.), the reaction mixture was filtered and distilled the reaction mixture to obtain the crude product. Yield: 0.11 g

### Example 3

A mixture of isobutylbenzene (1.5mol) acetic anhydride (0.375mol) and microcrystalline zeolite beta-I catalyst (20 g) were stirred in a round bottomed flask (1 lit) under nitrogen atmosphere at 130 °C temperature. After completion of the reaction (followed by G. C.), the reaction mixture was filtered and distilled the reaction mixture to obtain the crude product. Yield: 13.4 g

### Example 4

A mixture of isobutylbenzene (40 mmol), acetic anhydride (10 mmol) and microcrystalline zeolite beta-II catalyst (0.5 g) were stirred in a round bottomed flask (50 ml) under nitrogen atmosphere at 130 °C temperature. After completion of the reaction (followed by G. C.), the reaction mixture was filtered and distilled the reaction mixture to obtain the crude product. Yield: 0.42 g

### Example 5

A mixture of isobutylbenzene (40mmol) acetic anhydride (10 mmol) and H⁺-exchanged microcrystalline zeolite beta-I catalyst (0.5 g) were stirred in a round bottomed flask (50 ml) under nitrogen atmosphere at 130 °C temperature. After completion of the reaction (followed by G. C.), the reaction mixture was filtered and 'distilled the reaction mixture to obtain the crude product. Yield: 0.36 g

### Comparative Example 6

A mixture of isobutylbenzene (40mmol) acetic anhydride (10 mmol) and Fe³⁺-exchanged zeolite beta catalyst (0.5 g) were stirred in a round bottomed flask (50 ml) under nitrogen atmosphere at 130 °C temperature. After completion of the reaction (followed by G. C.), the reaction mixture was filtered and distilled the reaction mixture to obtain the crude product. Yield: 0.21 g.

### Comparative Example 7

A mixture of isobutylbenzene (40mmol), acetic anhydride (10 mmol) and La³⁺-exchanged zeolite beta catalyst (0.5 g) were stirred in a round bottomed flask (50 ml) under nitrogen atmosphere at 130 °C temperature. After completion of the reaction (followed by G. C.), the reaction mixture was filtered and distilled the reaction mixture to obtain the crude product. Yield: 0.26 g

### Example 8

A mixture of isobutylbenzene (40mmol), acetic anhydride (10 mmol) and Ce³⁺-exchanged microcrystalline zeolite beta-II catalyst (0.5 g) were stirred in a round bottomed flask (50 ml) under nitrogen atmosphere at 130 °C temperature. After completion of the reaction (followed by G. C.), the reaction mixture was filtered and distilled the reaction mixture to obtain the crude product. Yield: 0.52 g.

**Table 1**

| Acylation of isobutyl benzene with acetic anhydride by various zeolite beta catalysts. | | | |
|---|---|---|---|
| Example No. | Catalyst | Time (h) | Isolated yield (%) |
| 2 (Comparative) | Zeolite beta | 8 | 6 |
| 3 | Microcrystalline beta-I | 6 | 20 |
| 4 | Microcrystalline beta-II | 3 | 25 |
| 5 | H⁺microcrystalline beta-I | 6 | 20 |
| 6 (Comparative) | Fe³⁺-beta | 8 | 12 |
| 7 (Comparative) | La³⁺-beta | 6 | 15 |
| 8 | Ce³⁺-micro crystalline beta-II | 3 | 30 |

The main advantages of the present Invention are:
1. A novel and ecofriendly process for the preparation of 4'-isobutylacetophenone.
2. The present process eliminates the use of corrosive and stoichiometric quantities of aluminium chloride.
3. Nanocrystalline, microcrystalline and metal exchanged nano-and microcrystalline zeolite beta have been used as catalysts for the acylation of isobutylbenzene for the first time.
4. Work-up procedure is simple.
5. The present process envisages no disposal problem as the catalyst can be used for several cycles. The catalyst was subjected to 4 recycles which displayed consistent activity.
6. The present process is environmentally safe since there is no disposal problem.
7. The process is economical.

## Claims

1. A process for the preparation of 4'-isobutylacetophenone from isobutylbenzene which comprises reacting isobutyl benzene with acetic anhydride as an acylating agent in the presence of a zeolite beta catalyst at a temperature ranges between 60 to 165°C for 2-24 h separating the catalyst by filtration from the reaction mixture and recovering the product by a conventional method, wherein the zeolite beta catalyst is selected from nano crystalline, microcrystalline and metal exchanged nano- and microcrystalline zeolite beta and wherein the particle size of nanocrystalline zeolite beta is 10 nm to 100 nm and the particle size of microcrystalline zeolite beta is 1 µm to 50 µm.

2. A process as claimed in claim 1 wherein metal ions selected for exchange are Ce³⁺, Zn²⁺, Fe³⁺, and La²⁺.

3. A process as claimed in claim 1 wherein the reaction is effected at a temperature in the range of 60 to 165°C for 2-12 hrs.

## Patentansprüche

1. Verfahren zur Herstellung von 4'-Isobutylacetophenon aus Isobutylbenzol, das Folgendes aufweist, nämlich Reagieren von Isobutylbenzol mit Essigsäureanhydrid als Acylierungsmittel in Gegenwart eines Beta-Zeolith-Katalysators in einem Temperaturbereich von 60 bis 165°C für 2 bis 24 Stunden, Abtrennen des Katalysators von dem Reaktionsgemisch durch Filtration und Aufbereiten des Produkts durch ein konventionelles Verfahren, wobei der Beta-Zeolith-Katalysator ausgewählt ist aus nanokristallinem, mikrokristallinem und metallausgetauschtem nano- und mikrokristallinem Beta-Zeolith und wobei die Teilchengröße des nanokristallinen Beta-Zeoliths bei 10 nm bis 100 nm und die Teilchengröße des mikrokristallinen Beta-Zeoliths bei 1 µm bis 50 µm liegt.

2. Verfahren nach Anspruch 1, wobei die für den Austausch ausgewählten Metallionen Ce³⁺, Zn²⁺, Fe³⁺ und La²⁺ sind.

3. Verfahren nach Anspruch 1, wobei die Reaktion bei einer Temperatur im Bereich von 60 bis 165°C für 2 bis 12 Stunden durchgeführt wird.

## Revendications

1. Procédé de préparation de la 4'-isobutylacétophénone à partir de l'isobutylbenzène, qui consiste à faire réagir de l'isobutylbenzène sur de l'anhydride acétique comme agent d'acylation, en la présence d'un catalyseur à base d'un zéolite bêta, dans un domaine de température compris entre 60 et 165° C et pendant 2 à 24 heures, à séparer le catalyseur par filtration du mélange réactionnel et à recueillir le produit par un procédé habituel, le catalyseur à base de zéolite bêta étant choisi parmi du zéolite bêta nanocristallin, du zéolite bêta microcristallin, du zéolite bêta nanocristallin ayant subi un échange de métal et du zéolite bêta microcristallin ayant subi un échange de métal et la dimension de particule du zéolite bêta nanocristallin étant comprise entre 10 nm et 100 nm et la dimension de particule du zéolite bêta microcristallin étant comprise entre 1 µm et 50 µm.

2. Procédé suivant la revendication 1, dans lequel les ions métalliques sélectionnés pour l'échange sont Ce³⁺, Zn²⁺, Fe³⁺, La²⁺.

3. Procédé suivant la revendication 1, dans lequel on effectue la réaction à une température de l'ordre de 60 à 165° C pendant 2 à 12 heures.
